(19) Europäisches Patentamt
European Patent Office
Office européen des brevets

(11) **EP 4 132 604 B1**

(12) **EUROPÄISCHE PATENTSCHRIFT**

(45) Veröffentlichungstag und Bekanntmachung des Hinweises auf die Patenterteilung:
**27.03.2024   Patentblatt 2024/13**

(21) Anmeldenummer: **22731527.2**

(22) Anmeldetag: **25.05.2022**

(51) Internationale Patentklassifikation (IPC):
***A61M 1/16*** *(2006.01)*

(52) Gemeinsame Patentklassifikation (CPC):
**A61M 1/1609;** A61M 2202/0498; A61M 2205/3306

(86) Internationale Anmeldenummer:
**PCT/EP2022/064245**

(87) Internationale Veröffentlichungsnummer:
**WO 2022/248571 (01.12.2022 Gazette 2022/48)**

(54) **OPTISCHER SENSOR ZUR BESTIMMUNG EINER DIALYSEDOSIS**

OPTICAL SENSOR FOR DETERMINING A DIALYSIS DOSE

CAPTEUR OPTIQUE POUR DÉTERMINER UNE DOSE DE DIALYSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.05.2021   DE 102021113519**

(43) Veröffentlichungstag der Anmeldung:
**15.02.2023   Patentblatt 2023/07**

(73) Patentinhaber: **B. Braun Avitum AG**
**34212 Melsungen (DE)**

(72) Erfinder: **JANIK, Waldemar**
**34212 Melsungen (DE)**

(74) Vertreter: **Winter, Brandl - Partnerschaft mbB**
**Alois-Steinecker-Straße 22**
**85354 Freising (DE)**

(56) Entgegenhaltungen:
**EP-A1- 2 397 167          WO-A1-2013/186357**
**DE-A1-102011 008 482**

EP 4 132 604 B1

**Beschreibung**

Technisches Gebiet

Die vorliegende Offenbarung betrifft eine optische Messvorrichtung zur nichtinvasiven Bestimmung einer Dialysedosis.

Hintergrund der Erfindung

Zur Beurteilung der Effizienz einer extrakorporalen Blutbehandlung, wie beispielsweise einer Hämodialyse, wird als Parameter der Kt/V-Wert herangezogen. Dabei steht K für die Clearance, t für die Therapiedauer und V für das patientenindividuelle Verteilungsvolumen von Harnstoff im Körper.
Für die Bestimmung des Kt/V-Wertes können verschiedene Modelle berücksichtigt werden. Die einfachste Formel zum Ermitteln des Kt/V- Wertes während einer Dialysetherapie berücksichtigt weder die Generation von Harnstoff im Patienten während der Therapie, noch den sogenannten Reboundeffekt. Zur Bestimmung des Kt/V-Wertes wird in diesem Fall die Vorschrift:

$$\frac{Kt}{V} = \ln\left(\frac{c_0}{c(t)}\right)$$

verwendet. Dabei steht Co für die Anfangsharnstoffkonzentration und c(t) für die Harnstoffkonzentration zu einem gegebenen Zeitpunkt t.
Das am häufigsten eingesetzte Modell ist das Single-Pool-Modell, welches die Harnstoffgeneration während der Therapie/des zeitlichen Verlaufs der extrakorporalen Blutbehandlung berücksichtigt. Dabei wird vereinfacht angenommen, dass Harnstoff lediglich in einem großen Verteilungsvolumen gelöst ist.
Im Vergleich zu dem obenstehenden Modell wird berücksichtigt, dass während der Therapie Harnstoff in dem Körper des Patienten produziert wird. Des Weiteren berücksichtigt das Modell, dass eine durch Ultrafiltration auftretende Konvektion zusätzlich Harnstoff entfernt. Zur Bestimmung des Kt/V-Wertes wird in diesem Fall die Vorschrift:

$$sp\frac{Kt}{V} = -\ln\left(-0,008 \times t + \frac{c(t)}{c_0}\right) + \left(4 - 3,5 \times \frac{c(t)}{c_0}\right) \times \frac{UF}{W}$$

verwendet. Dabei steht UF für das Ultrafiltrationsvolumen und W für das Gewicht des Patienten.
Des Weiteren existiert ein Modell, welches den Reboundeffekt berücksichtigt. In der Realität ist die Bewegung von Harnstoff durch den Körper nicht uneingeschränkt möglich, da Harnstoff sowohl im intra- als auch im extrazellulären und im intravasalen Raum vorliegt. Durch das Modell, das abweichend vom Single-Pool-Modell die Existenz dieser unterschiedlichen Räume berücksichtigt, kann ein sogenannter äquilibrierter Kt/V ermittelt werden. Das Zurückströmen von Harnstoff nach der Therapie aus Organen mit geringer Blutdurchströmung in den intravasalen Raum wird hierdurch beachtet.
Zur messtechnischen Bestimmung des Wertes gibt es im Wesentlichen drei Methoden. Durch die Entnahme von Patientenblut vor und nach der Blutbehandlung und labortechnische Bestimmung der Harnstoffkonzentration kann im Anschluss der Kt/V-Wert berechnet werden. Allerdings ist diese Methode mit hohen Kosten für Labor- und Personalaufwand sowie Verbrauchsmaterialien verbunden und liefert nur retrospektiv einen Kt/V-Wert, sodass sie für die tägliche Routine nicht praktikabel ist.
Die zweite Möglichkeit sieht vor, die Zusammensetzung der Dialysierflüssigkeit im Laufe einer Behandlung kurzzeitig zu ändern. Durch Messung der dialysierflüssigkeitsseitigen Leitfähigkeit vor und hinter dem Dialysator vor und während der Leitfähigkeitsänderung kann dann die Dialysance berechnet werden. Hierbei macht man sich zunutze, dass das Natriumion, welches maßgeblich zur Leitfähigkeit beiträgt, ähnliche Diffusionseigenschaften besitzt wie das Harnstoffmolekül. Vorteile dieser Methode sind, dass keine weiteren Kosten auftreten und eine beinahe kontinuierliche Messung während jeder Therapie möglich ist. Nachteilig ist jedoch, dass die Änderung der Zusammensetzung der Dialysierflüssigkeit erstens eine gewisse Zeit benötigt, sodass eine vollständig kontinuierliche Messung nicht gegeben ist, und zweitens unter Umständen zu einer ungewünschten Verfälschung der Natriumbilanz während der Dialyse führen kann.
Eine wesentlich elegantere Möglichkeit zur Kt/V-Bestimmung wird in "Estimation of delivered dialysis dose by on-line monitoring oft he ultraviolet absorbance in the spent dialysate, Uhlin,F.; Lindberg, LG.; Magnusson M. Am J Kidney Dis. 2003 May;41 (5): 1026-36 beschrieben. Hierbei wird an dem Dialysierflüssigkeitsausgang die Absorbance bestimmt und daraus der Kt/V-Wert berechnet. Eine darauf basierende Messvorrichtung ist der Adimeasensor.

Stand der Technik

EP 2 397 167 A1 offenbart eine Vorrichtung zur extrakorporalen Blutbehandlung mit einer Messeinrichtung zur Bestimmung einer Lumineszenz der durch einen Ablauf fließenden verbrauchten Dialysierflüssigkeit, wobei die Messeinrichtung ein Detektorsystem aufweist, welches eine Intensität einer durch Lumineszenz erzeugten elektromagnetischen Strahlung ermittelt.

WO 2013 / 186 357 A1 offenbarteine Vorrichtung zur Überwachung einer extrakorporalen Blutbehandlung eines Patienten, wobei eine Probe einer bei der extrakorporalen Blutbehandlung verwendeten Flüssigkeit mit linear polarisiertem Bestrahlungslicht in unterschiedlichen Polarisationsebenen bestrahlt wird.

DE 10 2011 008 482 A1 offenbart einen fotometrischen Ultraviolettsensor, welcher beim Monitoring der Konzentration von mehreren im Dialyseprozess zu entfernenden Toxinen oder deren Kombinationen eingesetzt wird.

EP2 163 271 beschreibt ein Verfahren zur Ermittlung eines Kt/V-Wertes. Hierbei wird mittels eines UV-Sensors die Absorption der verbrauchten Dialysierflüssigkeit gemessen. Die ermittelte Absorption wird dann von einem Computer ausgewertet und ausgegeben. Hierbei wird die UV-Strahlungsquelle mit einer konstant hohen Stromstärke beaufschlagt. Dies führt zu einer Eigenerwärmung der UV-Strahlungsquelle, wodurch es zu Intensitätsschwankungen der von der Strahlungsquelle emittierten Strahlung kommen kann. Zusätzlich führt eine hohe Stromstärke zu einer Alterung der UV-Strahlungsquelle und damit zu einer Verkürzung der Lebensdauer der UV-Strahlungsquelle.

Andere Verfahren zur Ermittlung des Kt/V-Wertes basieren, wie vorangegangen beschrieben, beispielsweise auf der Ermittlung der Leitfähigkeit der Dialysierflüssigkeit. Hierfür kann es nötig sein, die Zusammensetzung der Dialysierflüssigkeit, die Blutflussrate oder die Dialysierflüssigkeitsflussrate kurzzeitig zu ändern, um die aktuelle Dialysedosis bestimmen zu können.

Kurzbeschreibung der Erfindung

Die Aufgabe der Erfindung ist es also, die Nachteile aus dem Stand der Technik zu vermeiden oder wenigstens zu mildern. Insbesondere soll eine Dialyseeffizienz/Dialysedosis einer extrakorporalen Blutbehandlung mittels einer Strahlungsquelle, insbesondere mittels einer UV-Strahlungsquelle, und einer Detektionseinheit bestimmt werden, wobei die Lebensdauer der Strahlungsquelle verlängert sowie der Stromverbrauch der Strahlungsquelle reduziert werden soll.

Diese Aufgabe wird durch die erfindungsgemäße Vorrichtung zur Bestimmung einer Dialysedosis einer extrakorporalen Blutbehandlung gelöst.

Die Vorrichtung zur Bestimmung einer Dialyseeffizienz/Dialysedosis einer extrakorporalen Blutbehandlung beinhaltet eine Strahlungsquelle, eine Küvette, mindestens eine Detektionseinheit und eine Regel-/Steuereinheit.

Die Strahlungsquelle ist dafür vorgesehen und ausgebildet, eine Strahlung zu emittieren, welche in ihrer Intensität über die Anpassung einer Stromstärke, mit der die Strahlungsquelle beaufschlagt wird, gesteuert/geregelt wird.

Die Küvette ist vorzugsweise mit einem Dialysierflüssigkeitseinlass und einem Dialysierflüssigkeitsauslass versehen und dafür vorgesehen und ausgebildet, von der von der Strahlungsquelle emittierten Strahlung durchdrungen zu werden.

Die Detektionseinheit ist dafür vorgesehen und ausgebildet, die Intensität der Strahlung, nachdem sie die die Küvette passiert hat, als Messdetektor zu detektieren.

Die Regel-/Steuereinheit bestimmt aus der von der Detektionseinheit detektierte Intensität der Strahlung die Dialyseeffizienz/Dialysedosis. Außerdem ist die Regel-/Steuereinheit mit der Strahlungsquelle und der Detektionseinheit verbunden und dazu vorgesehen und ausgebildet, die Intensität der emittierten Strahlung durch Anpassung der Stromstärke über den zeitlichen Verlauf der extrakorporalen Blutbehandlung mindestens einmal, basierend auf einer Abnahme einer Konzentration harnpflichtiger Stoffe in der Dialysierflüssigkeit, zu reduzieren.

In anderen Worten verfügt die Vorrichtung zur Bestimmung der Dialyseeffizienz einer extrakorporalen Blutbehandlung über eine Strahlungsquelle, insbesondere über eine UV-Strahlungsquelle, welche Strahlung, insbesondere Licht, mit einer Wellenlänge im ultravioletten Bereich emittiert. Die Wellenlänge der emittierten Strahlung liegt vorzugsweise in dem Bereich von 100 nm bis 380 nm, besonders bevorzugt in dem Bereich von 250 nm bis 300 nm und beispielsweise bei 254 nm oder 280 nm. Die Intensität der von der Strahlungsquelle emittierten Strahlung wird mittels der Stromstärke, mit der die Strahlungsquelle betrieben/beaufschlagt wird, gesteuert. So kann beispielsweise durch eine Erhöhung der Stromstärke die Intensität der emittierten Strahlung erhöht und durch eine Verringerung der Stromstärke die Intensität der emittierten Strahlung verringert werden.

Die Küvette ist vorzugsweise als Durchflussküvette vorgesehen und ausgebildet. Über einen Dialysierflüssigkeitseinlass strömt Dialysierflüssigkeit in die Küvette. Durch einen in Strömungsrichtung der Dialysierflüssigkeit hinter dem Dialysierflüssigkeitseinlass vorgesehenen Dialysierflüssigkeitsauslass verlässt die Dialysierflüssigkeit die Küvette. Vorzugsweise sind sowohl der Dialysierflüssigkeitseinlass als auch der Dialysierflüssigkeitsauslass vorgesehen und ausgebildet, mit einem Schlauch oder einer Leitung verbunden zu werden. Die Küvette wird von der Strahlung durchdrungen, welche von der Strahlungsquelle emittiert wird und besteht beispielsweise aus Kunststoff, Glas oder Quarzglas.

Die Detektionseinheit ist vorzugsweise ein Fotodetektor und dafür vorgesehen und ausgebildet, die von der Strahlungsquelle emittierte Strahlung zu detektieren. In anderen Worten ist die Detektionseinheit so konfiguriert, dass sie Strahlung, welche die Wellenlänge der von der Strahlungsquelle emittierten Strahlung aufweist, in ihrer Intensität detektieren kann. In einer bevorzugten Ausführungsform wandelt die Detektionseinheit die Strahlung unter Verwendung des photoelektrischen Effekts in ein elektrisches Signal um. In einer weiteren bevorzugten Ausführungsform ändert sich ein elektrischer Widerstand in Abhängigkeit von der Intensität der einfallenden Strahlung.

Die Regel-/Steuereinheit erhält von der Detektionseinheit die ermittelte Intensität der detektierten Strahlung vorzugweise in Form eines elektrischen Signals. Anhand dieses Intensitätswertes der Strahlung bestimmt die Regel-/Steuereinheit die Dialyseeffizienz/Dialysedosis. Die Regel-/Steuereinheit steuert die Intensität der emittierten Strahlung der Strahlungsquelle durch Anpassung der Stromstärke während des zeitlichen Verlaufs der extrakorporalen Blutbehandlung, wobei die Stromstärke und damit die Intensität der emittierten Strahlung während des zeitlichen Verlaufs der extrakorporalen Blutbehandlung mindestens einmal reduziert wird. Diese Reduktion der emittierten Strahlung geschieht basierend auf der Abnahme der Konzentration harnpflichtiger Stoffe in der Dialysierflüssigkeit. Die Größe der Abnahme der Konzentration harnpflichtiger Stoffe in der Dialysierflüssigkeit wird in einer ersten bevorzugten Ausführungsform gemessen. In einer weiteren bevorzugten Ausführungsform wird die Größe der Abnahme der harnpflichtigen Stoffe in der Dialysierflüssigkeit über den zeitlichen Verlauf der extrakorporalen Blutbehandlung prädiziert.

In nochmals anderen Worten besteht der Kern der Erfindung darin, dass die Intensität der von der Strahlungsquelle emittierten Strahlung über den zeitlichen Verlauf der extrakorporalen Blutbehandlung mindestens einmal reduziert wird, wobei diese Reduktion auf der Basis der Konzentrationsverringerung an harnpflichtigen Stoffen in der Dialysierflüssigkeit erfolgt.

Eine solche Reduktion der Intensität der emittierten Strahlung hat zur Folge, dass die Strahlungsquelle über den zeitlichen Verlauf der extrakorporalen Blutbehandlung weniger stark belastet wird und sich damit die Lebensdauer der Strahlungsquelle verlängert. Zusätzlich kann der Stromverbrauch der Strahlungsquelle reduziert werden.

Bei der Bestimmung der Dialysedosis besteht außerdem kein Kontakt zu dem Dialysat oder zu dem Blut des Patienten, was die Handhabung erleichtert und Verunreinigungen verhindert.

Ein Weiterer Vorteil der Messmethode besteht darin, dass die Zusammensetzung der Dialysierflüssigkeit, die Blutflussrate oder die Dialysierflüssigkeitsflussrate zur Bestimmung der Dialysedosis nicht geändert werden muss und es sich damit um eine passive Messmethode handelt.

Des Weiteren ist der Einsatz einer Detektionseinheit mit einem größeren linearen Messbereich möglich, was das Messen höherer Absorbancewerte ermöglicht und somit genauere Kt/V-Werte bestimmt werden können.

In einem Aspekt der Vorrichtung ist die Regel-/Steuereinheit dafür vorgesehen und ausgebildet, die Strahlungsquelle derart zu steuern, dass die von der Detektionseinheit detektierte Intensität der Strahlung über den zeitlichen Verlauf der extrakorporalen Blutbehandlung konstant bleibt.

In anderen Worten wird die Stromstärke, mit der die Strahlungsquelle beaufschlagt wird, von der Regel-/Steuereinheit so gesteuert, dass nach einer Einregelphase die Intensität der Strahlung über den zeitlichen Verlauf der extrakorporalen Blutbehandlung, die an der Detektionseinheit detektiert wird, konstant gehalten wird.

Da über den Zeitlichen Verlauf der extrakorporalen Blutbehandlung die Konzentration an harnpflichtigen, strahlungsabsorbierenden Stoffen in der verbrauchten Dialysierflüssigkeit abnimmt und damit weniger der durch die Küvette hindurch von der Strahlungsquelle emittierten Strahlung von den harnpflichtigen Stoffen absorbiert wird, reduziert die Regel-/Steuereinheit die Stromstärke und damit die Intensität der emittierten Strahlung über den zeitlichen Verlauf der extrakorporalen Blutbehandlung, um die von der Detektionseinheit detektierte Intensität der Strahlung konstant zu halten.

Eine solche Regelung kann vorzugsweise mittels PI- oder PID-Reglern ausgebildet werden. Die detektierte Intensität der Strahlung an der Detektionseinheit zu dem Zeitpunkt nach der Einregelphase ($I(t3)$) ist dabei die Führungsgröße, die detektierte Intensität der Strahlung ($I(t)$) an der Detektionseinheit die Regelgröße und der Strom/die Stromstärke die Stellgröße.

In einem weiteren Aspekt der Vorrichtung wird die Stromstärke, mit der die Strahlungsquelle beaufschlagt wird, zur Bestimmung der Dialyseeffizienz/Dialysedosis ausgewertet/verwendet.

In anderen Worten wird die Stromstärke, mit der die Strahlungsquelle über den zeitlichen Verlauf der extrakorporalen Blutbehandlung beaufschlagt wird, vorzugsweise von der Regel-/Steuereinheit ausgewertet, um daraus die Dialyseeffizienz/Dialysedosis zu bestimmen.

In einem weiteren Aspekt der Vorrichtung handelt es sich bei der Dialyseeffizienz/Dialysedosis um den Kt/V-Wert, wobei K die Clearance, t die Therapiedauer und V das patientenindividuelle Verteilungsvolumen von Harnstoff in dem Körper beschreibt. Die Regel-/Steuereinheit ist dafür vorgesehen und ausgebildet, die Dialyseeffizienz/Dialysedosis über die Vorschrift $Kt/V = ln(I(t3)/I(t))$ zu bestimmen, wobei I die Stromstärke, t3 einen festen Zeitpunkt und t die Therapiedauer/Zeit beschreibt.

In anderen Worten wird der Kt/V-Wert über die Vorschrift

$$\frac{Kt}{V} = \ln\left(\frac{I(t3)}{I(t)}\right)$$

vorzugsweise in der Regel-/Steuereinheit ermittelt, mit K als Clearance, t als Therapiedauer und V als patientenindividuelles Verteilungsvolumen von Harnstoff in dem Körper des Patienten. Die detektierte Intensität der Strahlung an der Detektionseinheit zu dem Zeitpunkt nach der Einregelphase wird in Form einer Stromstärke detektiert und mit I(t3) bezeichnet und die detektierte Intensität der Strahlung an der Detektionseinheit wird ebenfalls in Form einer Stromstärke detektiert und mit I(t) bezeichnet.

In einem weiteren Aspekt der Vorrichtung handelt es sich bei der Dialyseeffizienz/Dialysedosis um den Kt/V-Wert, wobei K die Clearance, t die Therapiedauer und V das patientenindividuelle Verteilungsvolumen von Harnstoff im Körper beschreibt. Die Regel-/Steuereinheit ist dafür vorgesehen und ausgebildet, die Dialyseeffizienz/Dialysedosis über die Vorschrift Kt/V = -ln(-0,008*t+(I(t)/I(t3))+(4-3,5*(I(t)/I(t3))*UF/W zu bestimmen, wobei I die Stromstärke, t3 einen festen Zeitpunkt, t die Therapiedauer, UF das Ultrafiltrationsvolumen und W das Gewicht des Patienten beschreibt.

In anderen Worten wird der Kt/V-Wert über die Vorschrift

$$sp\frac{Kt}{V} = -\ln\left(-0{,}008 \times t + \frac{I(t)}{I(t3)}\right) + \left(4-3{,}5 \times \frac{I(t)}{I(t3)}\right) \times \frac{UF}{W}$$

vorzugsweise in der Regel-/Steuereinheit ermittelt, mit K als Clearance, t als Therapiedauer und V als patientenindividuelles Verteilungsvolumen von Harnstoff in dem Körper des Patienten. Die detektierte Intensität der Strahlung an der Detektionseinheit zu dem Zeitpunkt nach der Einregelphase wird in Form einer Stromstärke detektiert und mit I(t3) bezeichnet und die detektierte Intensität der Strahlung wird ebenfalls in Form einer Stromstärke detektiert und mit I(t) bezeichnet. Weiter beschreibt UF das Ultrafiltrationsvolumen und W das Gewicht des Patienten.

In einem weiteren Aspekt der Vorrichtung wird die ermittelte Stromstärke auf eine Referenztemperatur normiert.

In anderen Worten wird die Stromstärke, mit der die Strahlungsquelle beaufschlagt wird und von der Regel-/Steuereinheit zur Bestimmung der Dialyseeffizienz/Dialysedosis ausgewertet wird, vor der Auswertung auf eine Referenztemperatur normiert. Dies geschieht, da die von der Strahlungsquelle ausgestrahlte Intensität der Strahlung temperaturabhängig ist. Bei steigender Temperatur sinkt die Intensität der emittierten Strahlung bei konstantem Strom/konstanter Stromstärke. Vorzugsweise wird die Dialysierflüssigkeitstemperatur an einem Temperatursensor am Dialysierflüssigkeitsausgang der Dialysemaschine detektiert.

Bevorzugt wird der Strom auf die Referenztemperatur von 37 °C kompensiert. Dies geschieht besonders bevorzugt über die Vorschrift

$$I_{37\,°C} = I_T - \alpha \times (T - 37\ °C)$$

wobei $I_{37\,°C}$ für den auf 37 °C kompensierten Strom steht, $I_T$ für den Strom der bei der gemessenen Temperatur T detektiert wird und $\alpha$ einen Temperaturkoeffizienten beschreibt.

Neben einer Kompensation auf 37 °C ist auch eine Kompensation auf jede beliebige weitere Temperatur möglich.

In einem weiteren Aspekt weist die Vorrichtung eine weitere Detektionseinheit auf, die dafür vorgesehen und ausgebildet ist, die Intensität der von der Strahlungsquelle emittierten Strahlung als Referenzdetektionseinheit zu detektieren. Der Therapieverlauf weist mindestens eine Einregelphase und mindestens eine Messphase auf, wobei die Regel-/Steuereinheit die Intensität der emittierten Strahlung über den zeitlichen Verlauf der extrakorporalen Blutbehandlung mindestens einmal in der Einregelphase reduziert und die Regel-/Steuereinheit die Strahlungsquelle durch Anpassung der Stromstärke derart steuert, dass die von der Referenzdetektionseinheit detektierte Intensität der Strahlung während der Messphase konstant bleibt und die Intensität der Strahlung an der Messdetektionseinheit zur Ermittlung der Dialyseeffizienz/Dialysedosis während der Messphase ausgewertet wird.

In anderen Worten ist neben der als Messdetektionseinheit vorgesehenen und ausgebildeten Detektionseinheit eine weitere, als Referenzdetektionseinheit vorgesehene und ausgebildete Detektionseinheit in der Vorrichtung vorgesehen. Diese Referenzdetektionseinheit wird verwendet, um mögliche Intensitätsschwankungen der von der Strahlungsquelle emittierten Strahlung auszugleichen. Vorzugsweise wird die von der Strahlungsquelle emittierte Strahlung an einem in Strahlungsrichtung vor der Küvette vorgesehen Strahlungsteiler geteilt, so dass ein erster Teil der Strahlung die Küvette passiert und von der Messdetektionseinheit detektiert wird und ein zweiter Teil der Strahlung von der Referenzdetektionseinheit detektiert wird, ohne die Küvette zu passieren. Alternativ ist die Referenzdetektionseinheit so angebracht und positioniert, dass sie die von der Strahlungsquelle emittierte Strahlung direkt erfassen kann ohne dass die Strahlung die Küvette passiert. In diesem Fall kann der Strahlungsteiler entfallen.

Der Therapieverlauf ist in mindestens eine Einregelphase und mindestens eine Messphase unterteilt. Die Regel-/Steuereinheit ist dafür vorgesehen und ausgebildet, die Intensität der von der Strahlungsquelle emittierten Strahlung mindestens einmal in der Einregelphase zu reduzieren. Zusätzlich ist die Regel-/Steuereinheit dafür vorgesehen und ausgebildet, dass sie mittels Anpassung der Stromstärke, mit der die Strahlungsquelle beaufschlagt wird, die Strahlungsquelle derart steuert, dass die von der Referenzdetektionseinheit detektierte Strahlung während der Messphase konstant bleibt. Die Regel-/Steuereinheit ist weiterhin dafür vorgesehen und ausgebildet, die Intensität der Strahlung, die während der Messphase von der Messdetektionseinheit detektiert wird, zur Bestimmung der Dialyseeffizienz/Dialysedosis zu verwenden.

In einem weiteren Aspekt der Vorrichtung werden die Einregelphase und/oder die Messphase durch Therapieparameter ausgelöst.

In anderen Worten wird, falls sich Maschinenparameter und/oder Therapieparameter ändern, die Einregelphase und/oder die Messphase begonnen/ausgelöst. So kann beispielsweise bei einer Erhöhung des Dialysierflüssigkeitsflusses die Einregelphase mit einer Intensitätsreduzierung der emittierten Strahlung der Strahlungsquelle durchgeführt werden, da sich durch die Erhöhung des Dialysierflüssigkeitsflusses eine Verdünnung der Dialysierflüssigkeit hinsichtlich der Konzentration harnpflichtiger Stoffe bzw. lichtabsorbierender Stoffe ergibt.

In einem weiteren Aspekt der Vorrichtung werden die Einregelphase und die Messphase an definierten Zeitpunkten der extrakorporalen Blutbehandlung durchgeführt.

In anderen Worten wird vor Beginn der extrakorporalen Blutbehandlung festgelegt, an welchen Zeitpunkten der extrakorporalen Blutbehandlung die Einregelphase und die Messphase durchgeführt werden. Vorzugsweise werden mehr Einregelphasen und Messphasen zu Beginn der extrakorporalen Blutbehandlung durchgeführt als zum Ende der extrakorporalen Blutbehandlung. Weiterhin vorzugsweise werden nur zu Beginn und zum Ende der extrakorporalen Blutbehandlung Einregelphasen und Messphasen durchgeführt.

Durch eine Reduzierung insbesondere der Messzyklen kann die Lebensdauer der Strahlungsquelle verlängert werden und Energie gespart werden.

In einem weiteren Aspekt der Vorrichtung wird eine Trübung der Küvette durch eine Verhältnisänderung zwischen der an der Messdetektionseinheit detektierten Intensität der Strahlung und der an der Referenzdetektionseinheit detektierten Intensität der Strahlung bestimmt.

In anderen Worten wird ein Strom S1 an die Strahlungsquelle angelegt und aus der Intensität der detektierten Strahlung am Messdetektor M(S1) und der Intensität der Strahlung der detektierten Strahlung am Referenzdetektor R(S1) wird das Verhältnis r mit der Vorschrift

$$r = \frac{M(S1)}{R(S1)}$$

vorzugsweise in der Regel-/Steuereinheit berechnet. Dieses Verhältnis r wird gespeichert und vorzugsweise mit mindestens einem weiteren gespeicherten Verhältnis r verglichen. Wenn dann festgestellt wird, dass das Verhältnis r abgesunken ist, deutet dies auf eine Trübung der Küvette hin. Vorzugsweise wird ein entsprechender Hinweis an den Bediener ausgegeben.

Weiterhin vorzugsweise wird aus mehreren gespeicherten Verhältnissen r ein Mittelwert oder ein Medianwert gebildet, mit dem das aktuell ermittelte Verhältnis r verglichen wird.

Beispielsweise kann das Verhältnis r mit dem Mittelwert der vorangegangenen fünf ermittelten Verhältnisse r verglichen werden. Alternativ kann das Verhältnis r mit dem Medianwert der vorangegangenen fünf ermittelten Verhältnisse r verglichen werden.

Zusätzlich kann eine Trendanalyse des Verhältnisses r über einen längeren Zeitraum durchgeführt werden. Mittels einer solchen Trendanalyse sind Veränderungen an der Vorrichtung oder an weiteren Bauteilen identifizierbar.

In einem weiteren Aspekt der Vorrichtung werden die Stromstärken und die Dauer, mit denen die Strahlungsquelle mit den Stromstärken beaufschlagt werden gespeichert und mittels dieser gespeicherten Werte eine Prädiktion der (Rest-) Lebensdauer der Strahlungsquelle durchgeführt.

In anderen Worten werden Stromstärken und/oder Therapieanzahlen und/oder die kumulierte Messdauer (die Dauer, für die die Strahlungsquelle eingeschaltet war) gespeichert und daraus eine Vorhersage über die verbleibende Lebensdauer der Strahlungsquelle abgeleitet.

Dies ermöglicht es einem Bediener, vor dem Ausfall der Vorrichtung Ersatz/ein Ersatzteil anzufordern, sodass die Vorrichtung vor dem Ausfall ausgetauscht werden kann.

Optional kann die Prädiktion als Grafik an den Bediener ausgegeben werden. Weiterhin optional kann die Bestellung eines Ersatzes empfohlen werden oder halbautomatisch oder vollautomatisch durchgeführt werden. Eine halbautomatische oder vollautomatische Bestellung erfolgt beispielsweise über ein mit der Vorrichtung verbundenes Beschaffungssystem.

In einem weiteren Aspekt der Vorrichtung emittiert die Strahlungsquelle nicht während der gesamten Messphase Strahlung. Die Strahlung wird vorzugsweise in zyklischen Intervallen emittiert. Beispielsweise kann die Strahlungsquelle während einer Messphase für fünf oder mehr Minuten deaktiviert werden, um anschließend wieder für eine Messdauer von einigen Sekunden aktiviert zu werden.

In einem weiteren Aspekt kann die Küvette zwischen der Strahlungsquelle und der Messdetektionseinheit angeordnet sein.

In einem weiteren Aspekt kann die Stromstärke, mit der die Strahlungsquelle beaufschlagt wird, durch eine monoton fallende Funktion beschreibbar sein.

Kurzbeschreibung der Figuren

Fig. 1 ist eine Darstellung einer Vorrichtung gemäß einer ersten Ausführungsform der vorliegenden Erfindung;

Fig. 2 ist eine Darstellung zur Veranschaulichung der Einbindung einer erfindungsgemäßen Vorrichtung nach der ersten oder einer zweiten Ausführungsform in den Behandlungsaufbau der extrakorporalen Blutbehandlung.

Fig. 3 ist ein Diagramm, welches das Signal/Intensität der Strahlung an einer Messdetektionseinheit der ersten Ausführungsform über den zeitlichen Verlauf der extrakorporalen Blutbehandlung darstellt.

Fig. 4 ist ein Diagramm, welches den Strom/die Stromstärke, mit dem die Strahlungsquelle der ersten Ausführungsform über den zeitlichen Verlauf der extrakorporalen Blutbehandlung beaufschlagt wird, darstellt.

Fig. 5 ist eine Darstellung einer Vorrichtung gemäß einer zweiten Ausführungsform der vorliegenden Erfindung.

Fig. 6 ist ein Diagramm, welches die Konzentration an harnpflichtigen Stoffen sowohl in dem Blut als auch in der Dialysierflüssigkeit über den zeitlichen Verlauf der extrakorporalen Blutbehandlung darstellt.

Fig. 7 ist ein Diagramm, welches eine Vorrichtungsidentifizierung der zweiten Ausführungsform darstellt, wobei das Signal/die Intensität der Strahlung an der Messdetektionseinheit und an der Referenzdetektionseinheit über dem Strom/der Stromstärke aufgetragen ist.

Fig. 8 ist ein Diagramm, welches schematisch ein typisches Absorptionsspektrum einer Dialysierflüssigkeit darstellt. Hierbei ist die Absorption der Dialysierflüssigkeit über der Wellenlänge in nm aufgetragen.

Fig. 9 ist ein Diagramm, welches schematisch einen Verlauf des Signals der Messdetektionseinheit, des Signals der Referenzdetektionseinheit und des Stroms/der Stromstärke einer zweiten Ausführungsform über den zeitlichen Verlauf der extrakorporalen Blutbehandlung darstellt.

Fig. 10 ist ein Beispiel für ein Diagramm, welches einem Bediener der Dialysemaschine ausgegeben wird und die Prädiktion der Lebensdauer der Strahlungsquelle darstellt.

Fig. 11 ist ein Diagramm, welches schematisch einen Verlauf des Signals der Messdetektionseinheit, des Signals der Referenzdetektionseinheit und des Stroms/der Stromstärke einer zweiten Ausführungsform über den zeitlichen Verlauf der extrakorporalen Blutbehandlung darstellt, wobei lediglich am Anfang und am Ende des zeitlichen Verlaufs der Therapie jeweils eine Einregelphase und eine Messphase vorliegen.

Beschreibung der Ausführungsbeispiele

Nachstehend werden Ausführungsbeispiele der vorliegenden Erfindung auf der Basis der zugehörigen Figuren beschrieben.

Erstes Ausführungsbeispiel

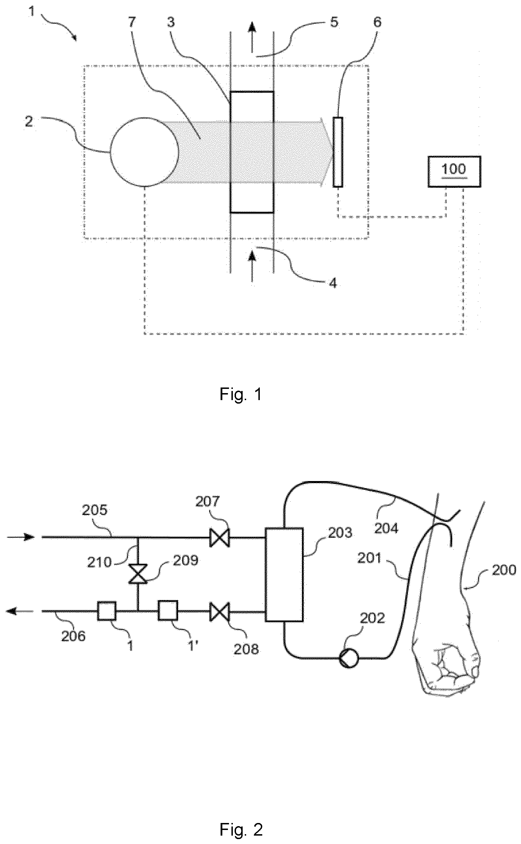

Fig. 1 zeigt eine erfindungsgemäße (Mess-) Vorrichtung 1, die aus einer Strahlungsquelle 2, insbesondere einer Lichtquelle, für im Wesentlichen monochromatische elektromagnetische Strahlung, einer Küvette 3, insbesondere einer Durchflussküvette, mit einem Dialysierflüssigkeitseinlass 4 und einem Dialysierflüssigkeitsauslass 5 sowie einer Detektionseinheit 6, insbesondere einem Fotodetektor, besteht. Die Detektionseinheit 6 ist dafür vorgesehen und ausgebildet, eine Strahlung 7, welche von der Strahlungsquelle 2 emittiert wird, in ihrer Intensität zu messen/detektieren.

Die Detektionseinheit 6 ist so in der Vorrichtung 1 vorgesehen und ausgebildet, dass sie die Intensität der von der Strahlungsquelle 2 emittierten/ausgestrahlten Strahlung 7 misst, nachdem diese die Küvette 3 passiert hat. Die Wellenlänge der emittierten Strahlung 7 der Strahlungsquelle 2 befindet sich im ultravioletten Bereich, beispielsweise bei 280 nm oder 254 nm. Die Intensität der von der Strahlungsquelle 2 emittierten Strahlung 7 wird von der Regel-/Steuereinheit 100 geregelt. Die Regel-/Steuereinheit 100 verarbeitet auch das Signal der Detektionseinheit 6.

Fig. 2 zeigt einen Behandlungsaufbau einer extrakorporalen Blutbehandlung. Während einer extrakorporalen Blutbehandlung wird Blut eines Patienten 200 über eine arterielle Leitung 201 mithilfe einer Fördereinheit 202, insbesondere einer Blutpumpe, zu einem Dialysator 203 gefördert, dort gereinigt und über eine venöse Leitung 204 zurück zu dem Patienten 200 geleitet. In entgegengesetzter Richtung zu dem Blutfluss strömt unverbrauchte Dialysierflüssigkeit aus einer Leitung 205 in den Dialysator 203. Die Dialysierflüssigkeit nimmt durch Diffusion und Konvektion harnpflichtige, (UV-)strahlungsabsorbierende Stoffe aus dem Blut des Patienten 200 auf und leitet diese harnpflichtigen strahlungsabsorbierenden Stoffe durch eine weitere Dialysierflüssigkeitsleitung 206 in Richtung eines Abflusses. Über eine Bypassleitung 210 kann die Dialysierflüssigkeit am Dialysator 203 vorbeigeleitet werden, indem ein Ventil 209 geöffnet ist und die Ventile 207 und 208 geschlossen sind. Stromabwärts des Dialysators 203, bezogen auf die Flussrichtung der Dialysierflüssigkeit befindet sich in der Dialysierflüssigkeitsleitung 206 die erfindungsgemäße Vorrichtung 1. Die Vorrichtung 1 kann in Flussrichtung der Dialysierflüssigkeit hinter (1 in Fig. 2) oder vor (1' in Fig. 2) der Mündungsstelle der Bypassleitung 210 in die Dialysierflüssigkeitsleitung 206 angebracht sein.

Durch die Stoffaustauschprozesse innerhalb des Dialysators 203 gelangen harnpflichtige Stoffe aus dem Blut in die Dialysierflüssigkeit. Manche dieser Stoffe absorbieren ultraviolette Strahlung/Licht, was von der erfindungsgemäßen Vorrichtung 1 detektiert werden kann. Dabei gilt, dass die gemessene Intensität der Strahlung 7 an der Detektionseinheit 6 umso niedriger ist, je höher die Konzentration an harnpflichtigen, strahlungsabsorbierenden Stoffen in der Dialysierflüssigkeit ist.

Im Laufe einer extrakorporalen Blutbehandlung nimmt die Konzentration an harnpflichtigen, strahlungsabsorbierenden Stoffen sowohl in dem Blut als auch in der verbrauchten Dialysierflüssigkeit allmählich ab, wodurch die Intensität der Strahlung 7 an der Detektionseinheit 6 steigt, falls die Intensität des von der Strahlungsquelle 2 ausgestrahlten Strahlung 7 konstant bleibt.

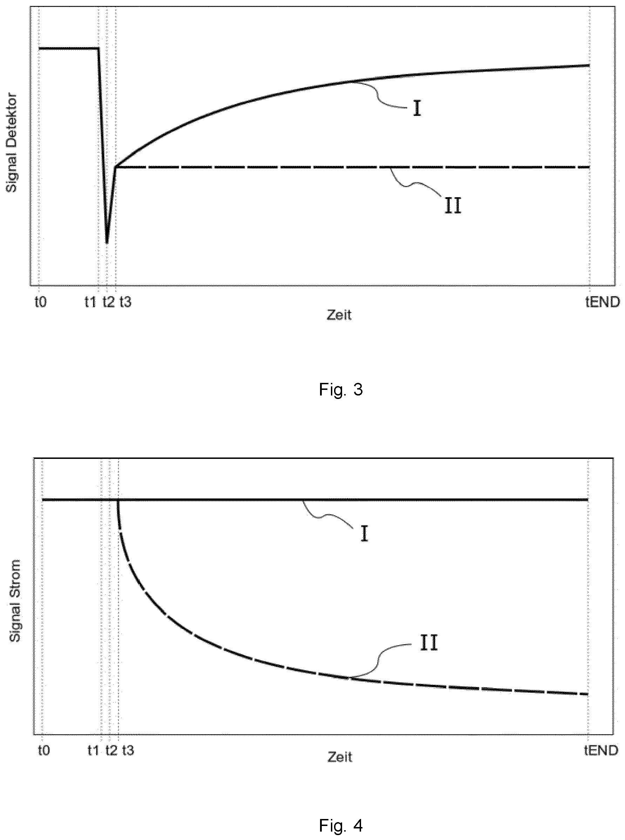

FIG. 3 zeigt schematisch den Signalverlauf an der Detektionseinheit 6 und FIG. 4 den dazugehörigen Strom/die dazugehörige Stromstärke zum Betreiben der Strahlungsquelle 2.

Zu dem Zeitpunkt t0 wird die Strahlungsquelle 2 mit einem vordefinierten, konstant hohen Strom betrieben. In dem Zeitraum t0 bis t1 befindet sich die Dialysemaschine im Bypass. Der Patient 200 wird währenddessen angelegt, was bedeutet, dass das Blut zuerst mit einer geringen Blutflussrate und anschließend mit einer bis zur Sollblutflussrate steigender Blutflussrate durch den Dialysator 203 zu fließen beginnt. Dadurch, dass während sich die Dialysemaschine im Bypass befindet die Dialysierflüssigkeit an dem Dialysator 203 vorbeifließt, befinden sich keine harnpflichtigen, strahlungsabsorbierenden Stoffe in der Dialysierflüssigkeit. Die gemessene Intensität an der Detektionseinheit 6 fällt demnach abhängig von dem Strom, mit dem die Strahlungsquelle beaufschlagt wird, ebenfalls hoch aus. Sobald der Sollblutfluss erreicht ist, wird der Bypassmodus deaktiviert und die eigentliche Dialyse/extrakorporale Blutbehandlung beginnt.

Während des Bypasses kommt es zu einer Ansättigung harnpflichtiger strahlungsabsorbierender Stoffe auf der Dialysierflüssigkeitsseite des Dialysators 203, welche durch die geschlossenen Ventile 207 und 208 begrenzt wird. Kurz nach Bypassende passiert die angesättigte Dialysierflüssigkeit die Vorrichtung 1, wodurch es zu einem Zeitpunkt t2 zu einem steilen Abfall des Messsignals/der detektierten Intensität der Strahlung an der Detektionseinheit 6 kommt. Sobald der Konzentrationsbolus die Vorrichtung 1 passiert hat, steigt das Messsignal/die detektierte Intensität der Strahlung 7 an der Detektionseinheit 6 wieder. Ein Zeitpunkt t3 bezeichnet dabei das Ende des Messsignals, welches von dem Konzentrationsbolus verursacht wurde.

Mit I wird in der Fig. 3 und in der Fig. 4 das Signal bezeichnet, welches an der Detektionseinheit 6 gemessen wird, falls die Stromstärke zum Betreiben der Strahlungsquelle 2 konstant gehalten wird.

Erfindungsgemäß wird nun aber nicht die Stromstärke konstant gehalten, sondern die detektierte Intensität der Strahlung 7 an der Detektionseinheit 6 ab dem Zeitpunkt t3. Dies geschieht durch Anpassung der Stromstärke. Hierfür können jedwede konventionellen Reglertypen zum Einsatz kommen, vorzugsweise PI- oder PID-Regler. Die detektierte Intensität der Strahlung 7 an der Detektionseinheit 6 zu dem Zeitpunkt t3 ist dabei die Führungsgröße, die detektierte Intensität der Strahlung 7 an der Detektionseinheit 6 die Regelgröße und die Stromstärke die Stellgröße.

Die erfindungsgemäßen Signale sind Fig. 3 und Fig. 4 mit II bezeichnet.

Zur Bestimmung des Kt/V-Wertes wird nun der Strom I herangezogen, mit welcher die Strahlungsquelle 2 betrieben wird:

$$\frac{Kt}{V} = \ln\left(\frac{I(t3)}{I(t)}\right)$$

Bzw.

$$sp\frac{Kt}{V} = -\ln\left(-0{,}008\times t + \frac{I(t)}{I(t3)}\right) + \left(4 - 3{,}5\times\frac{I(t)}{I(t3)}\right)\times\frac{UF}{W}$$

In dem zeitlichen Verlauf der Therapie/der extrakorporalen Blutbehandlung nimmt die Konzentration an harnpflichtigen, strahlungsabsorbierenden Stoffen in der verbrauchten Dialysierflüssigkeit ab, wodurch eine geringere Stromstärke benötigt wird, um die detektierte Intensität der Strahlung 7 an der Detektionseinheit 6 konstant zu halten.

Durch die allmähliche Absenkung der Stromstärke wird die Strahlungsquelle 2 weniger stark beansprucht, wodurch die Lebensdauer der Strahlungsquelle 2 verlängert wird.

Die Dauer des detektierten Signalverlaufs zwischen t1 und t3 kann abhängig von dem Dialysierflüssigkeitsfluss mit einer Dauer von 60 Sekunden bis 90 Sekunden als bekannt angenommen werden. Da t1 als Zeitpunkt der Deaktivierung des initialen Bypasses bekannt ist, ist somit auch t3 bekannt.

Die Zeitspanne t1 bis t3 ist im Vergleich zu der restlichen Therapiedauer/zu der Dauer der restlichen extrakorporalen Blutbehandlung sehr kurz. Vorzugsweise beträgt die Zeitspanne t1 bis t3 0,5 % der gesamten Therapiedauer.

Es ist bekannt, dass die von der Strahlungsquelle 2 ausgestrahlte Intensität der Strahlung 7 von der Temperatur abhängig ist. So sinkt die Intensität der Strahlung 7 für steigende Temperaturen bei konstantem Strom. Gängige Dialysemaschinen weisen am Dialysierflüssigkeitsausgang bereits einen Temperatursensor auf. Obwohl die Dialysierflüssigkeitstemperatur im Laufe einer Therapie recht konstant ist, kann dieser Temperatursensor verwendet werden, um einen möglichen Temperatureinfluss zu kompensieren. In der folgenden Gleichung wird der Strom auf einen Referenztemperaturwert von beispielsweise 37°C kompensiert:

$$I_{37\,°C} = I_T - \alpha\times(T - 37\,°C)$$

wobei $I_{37\,°C}$ für den auf 37 °C kompensierten Strom steht, $I_T$ für den Strom, der bei der gemessenen Temperatur T detektiert wird, und $\alpha$ einen Temperaturkoeffizienten darstellt.

Zweites Ausführungsbeispiel

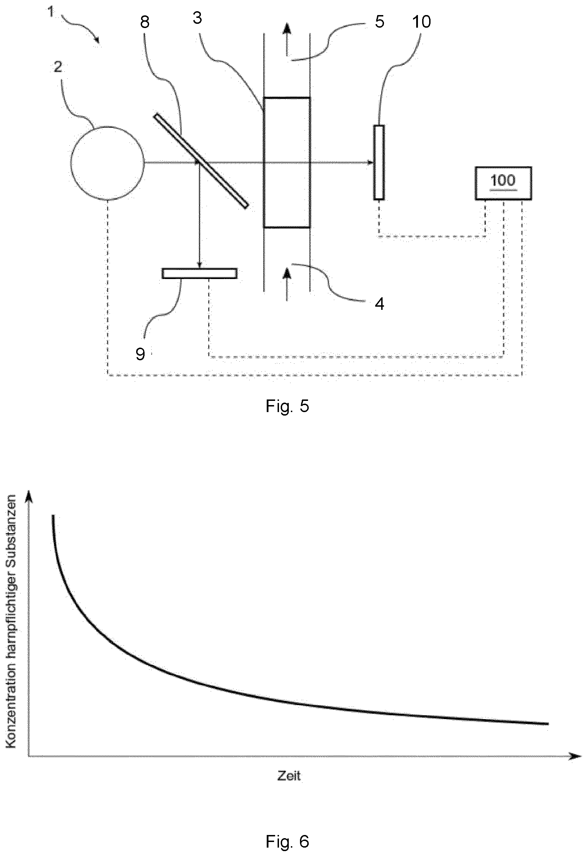

FIG. 5 zeigt eine erfindungsgemäße (Mess-)Vorrichtung 1, die aus einer Strahlungsquelle 2, insbesondere einer Lichtquelle, für im Wesentlichen monochromatische elektromagnetische Strahlung, einem halbdurchlässigen Strahlungsteiler 8, insbesondere einem Lichtteiler, einer Küvette 3, insbesondere einer Durchflussküvette, mit Dialysierflüssigkeitseinlass 4 und Dialysierflüssigkeitsauslass 5 sowie einer Referenzdetektionseinheit 9, insbesondere einem Referenzfotodetektor, und einer Messdetektionseinheit 10, insbesondere einem Messfotodetektor, besteht. Die Referenzdetektionseinheit 9 misst die Intensität der Strahlung, welche teilweise von dem Strahlungsteiler 8 reflektiert wird. Die Messdetektionseinheit 10 misst die Intensität der Strahlung, welche von der Strahlungsquelle 2 ausgestrahlt wird und anschließend die Küvette 3 passiert. Die Wellenlänge der ausgestrahlten Strahlung befindet sich im ultravioletten Bereich, beispielsweise bei 280 nm oder 254 nm. Die Intensität der ausgestrahlten Strahlung wird von der Regel-/Steuereinheit 100 durch Anpassung der Stromstärke geregelt. Die Regel-/Steuereinheit 100 verarbeitet auch das Signal der Referenzdetektionseinheit 9 und der Messdetektionseinheit 10.

Durch die Referenzdetektionseinheit 9 werden mögliche Intensitätsschwankungen der von der Strahlungsquelle 2 emittierten Strahlung ausgeglichen. Eine mögliche Ursache für solche Intensitätsschwankungen ist beispielsweise eine Temperaturänderung.

Der Strahlungsteiler 8 kann entfallen, wenn die Referenzdetektionseinheit 9 so positioniert ist, dass sie die von der Strahlungsquelle 2 emittierten Strahlung ebenfalls erfasst, ohne dass die Strahlung vorher die Küvette 3 passiert.

Fig. 2 zeigt einen Behandlungsaufbau einer extrakorporalen Blutbehandlung. Während einer extrakorporalen Blutbehandlung wird Blut eines Patienten 200 über eine arterielle Leitung 201 mithilfe einer Fördereinheit 202, insbesondere einer Blutpumpe, zu einem Dialysator 203 gefördert, dort gereinigt und über eine venöse Leitung 204 zurück zu dem Patienten 200 geleitet. In entgegengesetzter Richtung zu dem Blutfluss strömt unverbrauchte Dialysierflüssigkeit aus einer Leitung 205 in den Dialysator 203. Die Dialysierflüssigkeit nimmt durch Diffusion und Konvektion harnpflichtige, (UV-)strahlungsabsorbierende Stoffe aus dem Blut des Patienten 200 auf und leitet diese harnpflichtigen strahlungsabsorbierenden Stoffe durch eine weitere Dialysierflüssigkeitsleitung 206 in Richtung des Abflusses. Über eine Bypassleitung 210 kann die Dialysierflüssigkeit am Dialysator 203 vorbeigeleitet werden, indem ein Ventil 209 geöffnet ist und die Ventile 207 und 208 geschlossen sind. Stromabwärts des Dialysators, bezogen auf die Flussrichtung der Dialysierflüssigkeit, befindet sich in der Dialysierflüssigkeitsleitung 206 die erfindungsgemäße Vorrichtung 1. Die Vorrichtung 1 ist in Flussrichtung der Dialysierflüssigkeit hinter (1 in Fig. 2) oder vor (1' in Fig. 2) der Mündungsstelle der Bypassleitung 210 in die Dialysierflüssigkeitsleitung 206 angebracht.

In Fig. 6 ist dargestellt, dass durch die Stoffaustauschprozesse innerhalb des Dialysators 203 harnpflichtige, (UV-)strahlungsabsorbierende Stoffe aus dem Blut in die Dialysierflüssigkeit gelangen, wodurch die Konzentration an harnpflichtigen, strahlungsabsorbierenden Stoffen sowohl im Blut als auch in der Dialysierflüssigkeit im zeitlichen Verlauf der extrakorporalen Blutbehandlung allmählich abnimmt.

Es wurde bereits gezeigt, dass der zeitliche Abfall der Konzentration mit einer Absorbance/Absorptionsrate bzw. Extinktion in der Dialysierflüssigkeit korreliert.

Der folgende Ablauf beschreibt, wie die Absorbance bzw. Extinktion, die letztendlich zur Bestimmung des Kt/V-Werts benötigt wird, gemäß der Erfindung ermittelt werden kann.

1. Vorrichtungsidentifizierung SI

In dem ersten Schritt strömt frische, also unbelastete Dialysierflüssigkeit durch die Küvette 3. Dies ist zum Beispiel der Fall, wenn die Dialysierflüssigkeitsseite des Dialysators initial mit Dialysierflüssigkeit gespült wird. Dieses Spülen wird auch als Priming bezeichnet. Die Regel-/Steuereinheit 100 stellt einen ersten Strom/eine erste Stromstärke S1 zu dem Betreiben der Strahlungsquelle 2 ein. Vorzugsweise ist der Strom S1 so gewählt, dass die daraus resultierende Intensität der Strahlung der Strahlungsquelle 2 in dem Messbereich der Messdetektionseinheit 10 liegt.

Dadurch, dass der Strahlungsteiler 8 nur einen Teil der emittierten Strahlung in Richtung der Referenzdetektionseinheit 9 reflektiert, ist die bei dem Strom S1 gemessene Intensität der Strahlung an der Referenzdetektionseinheit 9 R(S1) kleiner als die gemessene Intensität der Strahlung an der Messdetektionseinheit 10 M(S1). Die Werte S1, R(S1) und M(S1) werden gespeichert.

In dem zweiten Schritt der Vorrichtungsidentifizierung wird der Strom auf den Wert S2 reduziert:

$$S2 = S1 \times \alpha$$

mit $\alpha < 1$, zum Beispiel $\alpha = 0{,}75$. S2 und die resultierenden Intensitätswerte an der Messdetektionseinheit 10 und an der Referenzdetektionseinheit 9 M(S2) und R(S2) werden ebenfalls gespeichert.

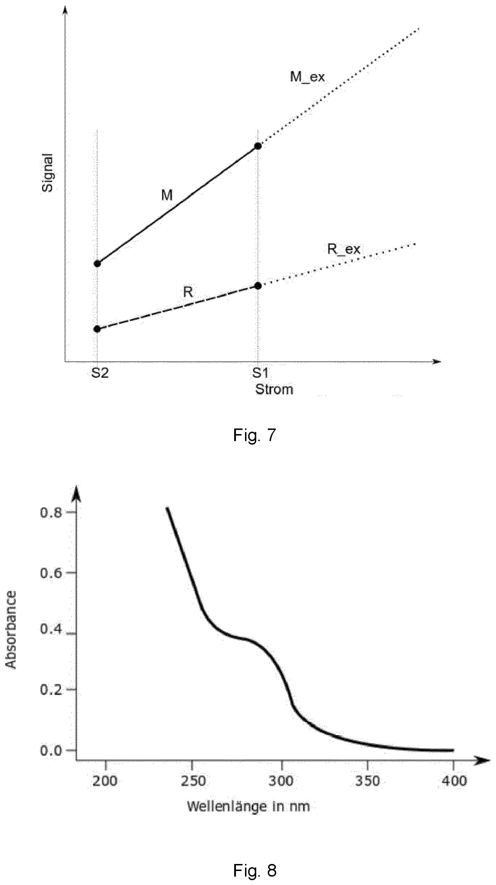

Da von einem linearen Zusammenhang zwischen Strom/Stromstärke und gemessenen Intensitätswerten der Strahlung ausgegangen werden kann, sind zwei Messpunkte für den nächsten Schritt ausreichend. Alternativ kann mindestens ein weiterer Stromwert eingestellt und die entsprechenden Intensitäten an der Referenzdetektionseinheit 9 und an der Messdetektionseinheit 10 gemessen und gespeichert werden. Anschließend werden, basierend auf den ermittelten Werten, die in Fig.7 dargestellten Kennlinien erstellt, welche die Intensität an der Referenzdetektionseinheit 9 und an der Messdetektionseinheit 10 in Abhängigkeit von der Stromstärke beschreiben:

$$M = a \times S + b$$

$$R = c \times S + d$$

Alternativ ist es denkbar, den Strom ab dem Wert S2 (quasi-)kontinuierlich bis zu einem Endwert zu reduzieren und alle Messwerte oder einen Teil der Messwerte in einer Look-up-Tabelle abzuspeichern.

Es ist außerdem denkbar, Polynome höheren Grades heranzuziehen, um die Beziehungen zwischen Strom und Intensitäten an der Referenzdetektionseinheit 9 und an der Messdetektionseinheit 10 zu beschreiben.

Weiterhin alternativ ist es möglich, zuerst einen kleinen Strom einzustellen und diesen anschließend zu erhöhen, statt zu reduzieren.

2. Erste Einregelphase EP1

Nachdem die Vorrichtung 1 identifiziert wurde, wird in einer ersten Einregelphase EP1 der Stromwert/die Stromstärke so lange angepasst, bis an der Messdetektionseinheit 10 ein erster Sollwert MSoll1 erreicht ist. Hierfür wird beispielsweise ein P-, PI-, oder PID-Regler verwendet.

Ein hierbei an der Referenzdetektionseinheit 9 detektierter Wert RSoll1 wird gespeichert. Vorzugsweise wird der Wert von der Regel-/Steuereinheit 100 gespeichert. Anschließend wird der Wert RSoll1 von der Regel-/Steuereinheit 100 durch Anpassung der Stromstärke konstant gehalten. Hierfür wird beispielsweise ein P-, PI-, oder PID-Regler verwendet.

### 3. Erste Messphase MP1

Sobald die Vorrichtung 1 eingeregelt ist, kann Blut des Patienten 200 durch den Dialysator 203 geleitet werden. Durch Diffusion und/oder Konvektion gelangen unter anderem harnpflichtige Substanzen auf die Dialysierflüssigkeitsseite. Manche dieser Substanzen absorbieren ultraviolette Strahlung. Fig. 8 zeigt schematisch ein typisches Absorptionsspektrum einer Dialysierflüssigkeit. Um Messungen an Flanken zu vermeiden, wird erfindungsgemäß eine Strahlungsquelle 2 gewählt, die Strahlung mit einer Wellenlänge von beispielsweise 280 nm emittiert. Die Absorbance in der ersten Messphase A1 wird gemäß:

$$A1 = \log\left(\frac{MSoll1}{M}\right)$$

bestimmt. MSoll1 ist hierbei der Sollwert aus der ersten Einregelphase und M der an der Messdetektionseinheit 10 gemessene Wert M.

Insbesondere zu Beginn einer Behandlung ist die Konzentration harnpflichtiger, strahlungsabsorbierender Substanzen in der verbrauchten Dialysierflüssigkeit recht hoch, wodurch der gemessene Wert M an der Messdetektionseinheit 10 unter Umständen gering ausfallen kann. Dies kann zu falschen Messergebnissen führen, da zum Beispiel nicht mehr zwischen Rauschen und wahrem Messwert unterschieden werden kann. Aus diesem Grund sieht die Erfindung alternativ vor, für den ersten Sollwert MSoll1 einen Wert Mex zu verwenden, welcher oberhalb des Messbereichs der Messdetektionseinheit 10 liegt. Dies kann, wie in Fig. 7 dargestellt, durch Extrapolation der in der Phase der Vorrichtungsidentifikation aufgenommen Werte geschehen. Der Sollwert kann einen beliebigen Wert annehmen, so lange gegeben ist, dass der dazugehörige Wert an der Referenzdetektionseinheit 9 Rex in dem Messbereich der Referenzdetektionseinheit 9 liegt, um weiterhin eine erfolgreiche Regelung gewährleisten zu können.

Dieses Vorgehen ermöglicht die Absorbancemessung bei höheren Konzentrationen strahlungsabsorbierender Stoffe, was einer Erhöhung des linearen Messbereichs der Vorrichtung 1 gleichkommt.

### 4. Zweite Einregelphase EP2

Wie vorstehend erwähnt, nimmt die Konzentration harnpflichtiger, strahlungsabsorbierender Substanzen in der Dialysierflüssigkeit im Laufe der extrakorporalen Blutbehandlung ab, wodurch die gemessene Intensität der Strahlung an der Messdetektionseinheit 10 tendenziell zunimmt, falls die von der Strahlungsquelle 2 emittierte Strahlung eine konstante Intensität aufweist. Gemäß der vorliegenden Erfindung wird die Intensität bzw. die Stromstärke bzw. der Sollwert an der Messdetektionseinheit 10 bzw. der Sollwert an der Referenzdetektionseinheit 9 deshalb mindestens einmal reduziert. Dies geschieht im einfachsten Fall durch Anwendung der in der Vorrichtungsidentifizierungsphase ermittelten Kennlinien:

$$RSoll2 = \frac{c}{a} \times (MSoll2 - b) + d$$

Befindet sich die Vorrichtung 1 in Flussrichtung der Dialysierflüssigkeit hinter der Mündungsstelle der Bypassleitung 210 in die Dialysierflüssigkeitsleitung 206, kann zur erneuten Einregelung der Vorrichtung 1 alternativ kurzzeitig in den Bypassmodus geschaltet werden. Da kurz nach Beginn des Bypasses reine Dialysierflüssigkeit durch die Vorrichtung 1 strömt, kann RSoll2 analog zu der ersten Einregelphase bestimmt werden.

### 5. Zweite Messphase MP2

In der zweiten Messphase wird die Absorbance analog zu der ersten Messphase bestimmt:

$$A2 = \log\left(\frac{MSoll2}{M}\right)$$

Bei weiteren Reduzierungen werden die Einregel- und Messphasen entsprechend wiederholt. Die Dauer der Einregelphasen ist im Vergleich zu den Messphasen deutlich geringer.

Während der Einregelphase werden keine Absorbancewerte bestimmt, die in die Bestimmung der Dialyseeffizienz (beispielsweise in den Kt/V) eingehen würden.

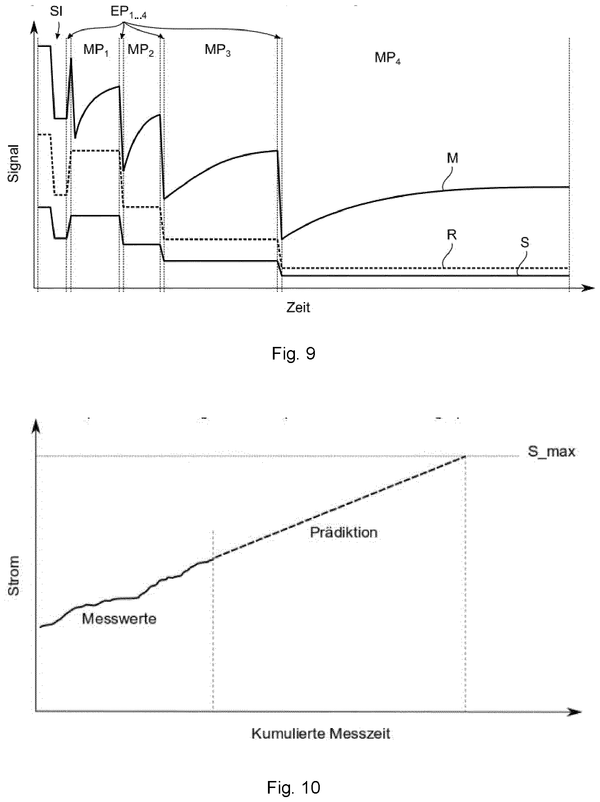

FIG. 9 zeigt beispielhaft den Verlauf der Signale der Messdetektionseinheit 10, der Referenzdetektionseinheit 9 und des Stroms im Laufe einer Therapie/einer extrakorporalen Blutbehandlung mit vier Reduzierungen der Intensität, bzw. des Stroms bzw. der Sollwerte. In den jeweiligen Messphasen wird das Signal an der Referenzdetektionseinheit 9 durch Anpassung des Stroms konstant gehalten.

Die Anzahl der Reduzierungen sowie die jeweilige Dauer der Messphasen ist variabel und kann beispielsweise voreingestellt sein.

Da aufgrund des in Fig. 6 dargestellten, näherungsweise exponentiell abklingenden Verlaufs der Konzentration harnpflichtiger, strahlungsabsorbierender Substanzen im Blut und in der Dialysierflüssigkeit mit einer schnelleren Reduzierung der Absorbance zu Beginn der Therapie gerechnet werden kann, bietet es sich an, mehr Intensitätsreduzierungen am Anfang statt am Ende der Therapie vorzunehmen.

Alternativ werden neue Einregel- und Messphasen begonnen, falls sich Parameter der Dialysemaschine oder der Behandlung ändern. Wird beispielsweise der Dialysierflüssigkeitsfluss von standardmäßig 500 ml/min auf 800 ml/min erhöht, kann ebenfalls eine Intensitätsreduzierung der von der Strahlungsquelle 2 emittierten Strahlung durchgeführt werden, da durch die Erhöhung des Dialysierflüssigkeitsflusses eine Verdünnung der Dialysierflüssigkeit hinsichtlich der Konzentration harnpflichtiger, strahlungsabsorbierender Stoffe verursacht wird. Ähnliches gilt für den extrakorporalen Blutfluss. Ist hingegen mit einer Erhöhung der Konzentration an harnpflichtigen, strahlungsabsorbierenden Stoffen zu rechnen (beispielsweise bei einer Dialysierflüssigkeitsflussabsenkung oder einer Blutflusserhöhung), kann die Intensität der von der Strahlungsquelle 2 emittierten Strahlung sogar erhöht werden.

Die Strahlungsquelle 2 muss nicht zwingend kontinuierlich Strahlung emittieren. Während einer Messphase kann sie beispielsweise auch für eine Dauer von fünf oder mehreren Minuten ausgeschaltet werden, um anschließend wieder für eine Messdauer von wenigen Sekunden eingeschaltet zu werden. Dieser Wechsel kann zyklisch erfolgen.

Die vorliegende Erfindung sieht vor, Stromwerte und Anzahl der Therapien und/oder die kumulierte Messzeit zu speichern und eine Vorhersage/Prädiktion über die verbleibende Lebensdauer der Strahlungsquelle 2 abzuleiten. Unter kumulierter Messzeit ist die Dauer zu verstehen, für die Strahlungsquelle 2 insgesamt eingeschaltet war.

Bei dem Stromwert handelt es sich beispielsweise um den Strom, der in der ersten Einregelphase benötigt wird, um an der Messdetektionseinheit 10 den Sollwert MSoll1 zu erreichen. Die Prädiktion kann genutzt werden, um rechtzeitig ein Ersatzteil anzufordern, sodass die Vorrichtung 1 noch vor einem Ausfall der Strahlungsquelle 2 ausgetauscht werden kann.

Die Grafik in Fig. 10 kann hierzu dem Bediener, insbesondere dem medizinischen Personal und/oder dem Technikpersonal und/oder dem Hersteller der Messvorrichtung zur Verfügung gestellt werden. Dies erfolgt vorzugweise durch Anzeige auf einem Bildschirm der Dialysevorrichtung oder durch Übermittlung zu einem Datenmanagementsystem. Eine Bestellung des Ersatzteils kann entweder empfohlen oder halb- bzw. vollautomatisch durchgeführt werden, falls die Anbindung beispielsweise an ein entsprechendes Beschaffungssystem vorhanden ist.

Alternativ ist die Vorrichtung so ausgebildet, dass die Strahlungsquelle 2 einzeln ersetzt werden kann.

Um einen möglichen Ausfall der Vorrichtung hinauszuzögern, wird erfindungsgemäß die Anzahl der Messzyklen reduziert.

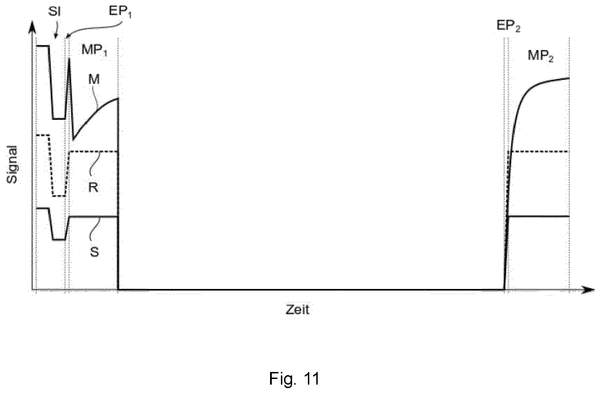

Fig. 11 stellt den Extremfall mit nur zwei Messphasen am Anfang und Ende einer Therapie dar, da zur Bestimmung des Kt/V-Werts mindestens der Anfangs- und Endwert der Absorbance gegeben sein muss.

Außerdem kann neben der Reduzierung der Messzyklen auch, wie zuvor beschrieben, zusätzlich eine Reduzierung der Intensität der von der Strahlungsquelle 2 emittierten Strahlung erfolgen.

Ein weiteres optionales Merkmal der Erfindung ist es, das Verhältnis r zwischen Wert der Messdetektionseinheit 10 und Wert der Referenzdetektionseinheit 9 während der Vorrichtungsidentifizierung auszuwerten, um mögliche Trübungen der Küvette 3 zu erkennen. Dies kann beim Anlegen eines Stroms S1 mit der Vorschrift:

$$r = \frac{M(S1)}{R(S1)}$$

erfolgen.

Das Verhältnis r wird über mehrere Vorrichtungsidentifizierungsphasen hinweg gespeichert. Stellt sich heraus, dass das Verhältnis r in einer Vorrichtungsidentifizierungsphase signifikant niedriger ist als beispielsweise der Mittelwert oder Median der beispielsweise fünf vorausgegangenen Vorrichtungsidentifizierungen, deutet dies auf eine Trübung der Küvette 3 hin, was beispielsweise dadurch hervorgerufen wurde, dass die Maschine nach der letzten Behandlung nicht oder nicht ausreichend desinfiziert und entkalkt wurde. Ein entsprechender Hinweis wird vorzugsweise auf dem Bildschirm der Dialysemaschine ausgegeben und eine Desinfektion durch einen Bediener durchgeführt. Alternativ erfolgt die Ausgabe als akustisches Signal oder das Aufleuchten einer Warnlampe.

Eine Trendanalyse über einen längeren Zeitraum, beispielsweise seit der ersten Inbetriebnahme der Vorrichtung, kann ebenfalls durchgeführt werden, um mögliche schleichende Veränderungen an den Bauteilen der Vorrichtung zu erkennen. Ändert sich das Verhältnis r über einen längeren Zeitraum und bewirkt eine Desinfektion bzw. Entkalkung der Dialysemaschine dagegen keine Änderung von r, so kann dies darauf hindeuten, dass sich das Ansprechverhalten der Messdetektionseinheit 10 und/oder der Referenzdetektionseinheit 9 verschlechtert. Auch hier ist es denkbar, dass eine frühzeitige Ersatzteilanforderung ausgelöst wird.

Da die Intensität der von der Strahlungsquelle 2 emittierten Strahlung, welche von der Messdetektionseinheit 10 und der Referenzdetektionseinheit 9 gemessen wird, temperaturabhängig ist, kann eine Temperaturkompensation der Messwerte bzw. des Stroms erfolgen. Dafür wird die Temperatur herangezogen, die von einem dialysierflüssigkeitsseitigen Temperatursensor stromab des Dialysators 203 oder von einem in der Vorrichtung 1 integrierten Temperatursensor gemessen wird. Es bietet sich eine Kompensation auf eine Temperatur von 37°C an, da dies die übliche Temperatur der Dialysierflüssigkeit ist.

Bezugszeichenliste

Vorrichtung 1
Strahlungsquelle 2
Küvette 3
Dialysierflüssigkeitseinlass 4
Dialysierflüssigkeitsauslass 5
Detektionseinheit 6
Strahlung 7
Strahlungsteiler 8
Referenzdetektionseinheit 9
Messdetektionseinheit 10
Regel-/Steuereinheit 100
Patient 200
Arterielle Leitung 201
Fördereinheit 202
Dialysator 203
Venöse Leitung 204
Leitung 205
Dialysierflüssigkeitsleitung 206
Ventil 207
Ventil 208
Ventil 209
Bypassleitung 210

**Patentansprüche**

1. Vorrichtung (1) zur Bestimmung einer Dialyseeffizienz/Dialysedosis einer extrakorporalen Blutbehandlung mit einer Strahlungsquelle (2), einer Küvette (3), mindestens einer Detektionseinheit (6, 10) und einer Regel-/Steuereinheit (100), wobei

die Strahlungsquelle (2) dafür vorgesehen und ausgebildet ist, eine Strahlung (7) zu emittieren, welche in ihrer Intensität über die Anpassung einer Stromstärke, mit der die Strahlungsquelle (2) beaufschlagt wird, gesteuert/geregelt wird,
die Küvette (3) vorzugsweise mit einem Dialysierflüssigkeitseinlass (4) und einem Dialysierflüssigkeitsauslass (5) versehen ist und dafür vorgesehen und ausgebildet ist, von der von der Strahlungsquelle (2) emittierten Strahlung (7) durchdrungen zu werden,
die Detektionseinheit (6, 10) dafür vorgesehen und ausgebildet ist, die Intensität der Strahlung (7), nachdem sie die die Küvette passiert hat, als Messdetekionseinheit zu detektieren,
die Regel-/Steuereinheit (100) aus der von der Detektionseinheit (6, 10) detektierten Intensität der Strahlung (7) die Dialyseeffizienz/Dialysedosis bestimmt,
**dadurch gekennzeichnet, dass**
die Regel-/Steuereinheit (100) mit der Strahlungsquelle (2) und der Detektionseinheit (6, 10) verbunden ist und dazu vorgesehen und ausgebildet ist, die Intensität der emittierten Strahlung (7) durch Anpassung der Stromstärke über den zeitlichen Verlauf der extrakorporalen Blutbehandlung mindestens einmal, basierend auf einer Abnahme einer Konzentration harnpflichtiger Stoffe in der Dialysierflüssigkeit, zu reduzieren.

2. Vorrichtung nach Anspruch 1, **dadurch gekennzeichnet, dass** die Regel-/Steuereinheit (100) dazu vorgesehen und ausgebildet ist, die Strahlungsquelle (2) derart zu steuern, dass die von der Detektionseinheit (6, 10) detektierte Intensität der Strahlung (7) über den zeitlichen Verlauf der extrakorporalen Blutbehandlung konstant bleibt.

3. Vorrichtung nach Anspruch 2, **dadurch gekennzeichnet, dass** die Stromstärke, mit der die Strahlungsquelle (2) beaufschlagt wird, zur Bestimmung der Dialyseeffizienz/Dialysedosis ausgewertet/verwendet wird.

4. Vorrichtung nach Anspruch 3, **dadurch gekennzeichnet, dass** es sich bei der Dialyseeffizienz/Dialysedosis um den Kt/V-Wert handelt, wobei K die Clearance, t die Therapiedauer und V das patientenindividuelle Verteilungsvolumen von Harnstoff im Körper beschreibt, und die Regel-/Steuereinheit (100) dafür vorgesehen und ausgebildet ist, die Dialyseeffizienz/Dialysedosis über die Vorschrift $Kt/V = \ln(I(t3)/I(t))$ zu bestimmen, wobei I die Stromstärke, t3 einen festen Zeitpunkt und t die Therapiedauer beschreibt.

5. Vorrichtung nach Anspruch 3, **dadurch gekennzeichnet, dass** es sich bei der Dialyseeffizienz/Dialysedosis um den Kt/V-Wert handelt, wobei K die Clearance, t die Therapiedauer und V das patientenindividuelle Verteilungsvolumen von Harnstoff im Körper beschreibt, und die Regel-/Steuereinheit (100) dafür vorgesehen und ausgebildet ist, die Dialyseeffizienz/Dialysedosis über die Vorschrift $Kt/V = -\ln(-0,008*t+(I(t)/I(t3))+(4-3,5*(I(t)/I(t3))*UF/W$ zu bestimmen, wobei I die Stromstärke, t3 einen festen Zeitpunkt, t die Therapiedauer, UF das Ultrafiltrationsvolumen und W das Gewicht des Patienten (200) beschreibt.

6. Vorrichtung nach einem der Ansprüche 3 bis 5, **dadurch gekennzeichnet, dass** die ermittelte Stromstärke auf einen Referenztemperaturwert normiert wird.

7. Vorrichtung nach Anspruch 1, wobei die Vorrichtung eine weitere Detektionseinheit (9) aufweist, die dafür vorgesehen und ausgebildet ist, die Intensität der von der Strahlungsquelle emittierten Strahlung als Referenzdetektionseinheit (9) zu detektieren, **dadurch gekennzeichnet, dass** der Therapieverlauf mindestens eine Einregelphase und mindestens eine Messphase aufweist, wobei die Regel-/Steuereinheit (100) die Intensität der emittierten Strahlung (7) über den zeitlichen Verlauf der extrakorporalen Blutbehandlung mindestens einmal in der Einregelphase reduziert und die Regel-/Steuereinheit (100) die Strahlungsquelle (2) durch Anpassung der Stromstärke derart steuert, dass die von der Referenzdetektionseinheit (9) detektierte Intensität der Strahlung (7) während der Messphase konstant bleibt und die Intensität der Strahlung (7) an der Messdetektionseinheit (10) zur Ermittlung der Dialyseeffizienz/Dialysedosis während der Messphase ausgewertet wird.

8. Vorrichtung nach Anspruch 7, **dadurch gekennzeichnet, dass** die Einregelphase und/oder die Messphase durch Therapieparameter ausgelöst werden.

9. Vorrichtung nach Anspruch 7, **dadurch gekennzeichnet, dass** die Einregelphase und die Messphase an definierten Zeitpunkten der extrakorporalen Blutbehandlung durchgeführt werden.

10. Vorrichtung nach einem der Ansprüche 7 bis 9, **dadurch gekennzeichnet, dass** eine Trübung der Küvette (3) durch eine Verhältnisänderung zwischen der an der Messdetektionseinheit (10) detektierten Intensität der Strahlung (7) und der an der Referenzdetektionseinheit (9) detektierten Intensität der Strahlung (7) bestimmt wird.

11. Vorrichtung nach einem der Ansprüche 1 bis 10, **dadurch gekennzeichnet, dass** die Stromstärken und die Dauer, mit denen die Strahlungsquelle (2) mit den Stromstärken beaufschlagt werden, gespeichert werden und mittels dieser gespeicherten Werte eine Prädiktion der (Rest-) Lebensdauer der Strahlungsquelle (2) durchgeführt wird.

12. Vorrichtung nach einem der Ansprüche 1 bis 11, **dadurch gekennzeichnet, dass** die Küvette (3) zwischen der Strahlungsquelle (2) und der Messdetektionseinheit (10) angeordnet ist.

**Claims**

1. A device (1) for determining a dialysis efficiency/dialysis dose of an extracorporeal blood treatment with a radiation source (2), a cuvette (3), at least one detection unit (6, 10) and a regulation/control unit (100), wherein

   the radiation source (2) is intended and configured to emit a radiation (7), the intensity of which is controlled/regulated by adjusting a current strength to which the radiation source (2) is subjected,
   the cuvette (3) is preferably provided with a dialysis fluid inlet (4) and a dialysis fluid outlet (5) and is intended and configured to be penetrated by the radiation (7) emitted by the radiation source (2),
   the detection unit (6, 10) is provided and adapted to detect, as a measurement detection unit, the intensity of the radiation (7) after it has passed the cuvette,
   the regulation/control unit (100) determines the dialysis efficiency/dialysis dose from the intensity of the radiation (7) detected by the detection unit (6, 10),
   **characterized in that**
   the regulation/control unit (100) is connected to the radiation source (2) and the detection unit (6, 10) and is provided and configured to reduce the intensity of the emitted radiation (7) by adjusting the current strength over the time course of the extracorporeal blood treatment at least once, based on a decrease in a concentration of urinary substances in the dialysis fluid.

2. The device according to claim 1, **characterized in that** the regulation/control unit (100) is provided and configured to control the radiation source (2) in such a way that the intensity of the radiation (7) detected by the detection unit (6, 10) remains constant over the time course of the extracorporeal blood treatment.

3. The device according to claim 2, **characterized in that** the current strength applied to the radiation source (2) is evaluated/used for determining the dialysis efficiency/dialysis dose.

4. The device according to claim 3, **characterized in that** the dialysis efficiency/dialysis dose is the Kt/V value, where K describes the clearance, t the therapy duration and V the patient-specific distribution volume of urea in the body, and the regulation/control unit (100) is provided and configured to determine the dialysis efficiency/dialysis dose via the rule Kt/V = ln(I(t3)/I(t)), where I describes the current strength, t3 a fixed point in time and t the therapy duration.

5. The device according to claim 3, **characterized in that** the dialysis efficiency/dialysis dose is the Kt/V value, where K describes the clearance, t the therapy duration and V the patient-specific distribution volume of urea in the body, and the regulation/control unit (100) is provided and configured to determine the dialysis efficiency/dialysis dose via the rule Kt/V = -ln(-0.008*t+(I(t)/I(t3))+(4-3.5*(I(t)/I(t3))*UF/W, where I describes the current strength, t3 describes a fixed point in time, t describes the therapy duration, UF describes the ultrafiltration volume and W describes the weight of the patient (200).

6. The device according to one of claims 3 to 5, **characterized in that** the determined current strength is normalized to a reference temperature value.

7. The device according to claim 1, wherein the device comprises a further detection unit (9) provided and configured to detect the intensity of the radiation emitted from the radiation source as a reference detection unit (9), **characterized in that** the course of therapy comprises at least one regulation phase and at least one measuring phase, wherein the regulation/control unit (100) reduces the intensity of the emitted radiation (7) over the time course of the extracorporeal blood treatment at least once in the regulation phase and the regulation/control unit (100) controls the radiation source (2) by adjusting the current strength in such a way that the intensity of the radiation (7) detected by the reference detection unit (9) remains constant during the measuring phase and the intensity of the radiation (7) is evaluated at the measuring detection unit (10) for determining the dialysis efficiency/dialysis dose during the measuring phase.

8. The device according to claim 7, **characterized in that** the regulation phase and/or the measuring phase are triggered by therapy parameters.

9. The device according to claim 7, **characterized in that** the regulation phase and the measuring phase are performed at defined times of the extracorporeal blood treatment.

10. The device according to one of claims 7 to 9, **characterized in that** an opacity of the cuvette (3) is determined by a ratio change between the intensity of the radiation (7) detected at the measuring detection unit (10) and the intensity of the radiation (7) detected at the reference detection unit (9).

11. The device according to one of claims 1 to 10, **characterized in that** the current strengths and the duration with which the radiation source (2) is subjected to the current strengths are stored and a prediction of the (residual) lifetime of the radiation source (2) is carried out via these stored values.

12. The device according to one of claims 1 to 11, **characterized in that** the cuvette (3) is arranged between the radiation source (2) and the measuring detection unit (10).

**Revendications**

1. Dispositif (1) destiné à la détermination d'une efficacité de dialyse/dose de dialyse d'un traitement extracorporel du sang avec une source de rayonnement (2), une cuvette (3), au moins une unité de détection (6, 10) et une unité de régulation/commande (100), dans lequel

la source de rayonnement (2) est prévue et réalisée pour émettre un rayonnement (7), dont l'intensité est commandée/réglée par le biais de l'adaptation d'un ampérage avec lequel la source de rayonnement (2) est alimentée,
la cuvette (3) est de préférence dotée d'une entrée de liquide de dialyse (4) et d'une sortie de liquide de dialyse (5) et est prévue et réalisée pour être traversée par le rayonnement (7) émis par la source de rayonnement (2),
l'unité de détection (6, 10) est prévue et réalisée pour détecter en tant qu'unité de détection de mesure l'intensité du rayonnement (7) une fois qu'il a dépassé la cuvette,
l'unité de régulation/commande (100) détermine l'efficacité de dialyse/la dose de dialyse à partir de l'intensité du rayonnement (7) détectée par l'unité de détection (6, 10),
**caractérisé en ce que**
l'unité de régulation/commande (100) est reliée à la source de rayonnement (2) et à l'unité de détection (6, 10) et est prévue et réalisée de manière à réduire l'intensité du rayonnement émis (7) par le biais de l'adaptation de l'ampérage pendant l'évolution dans le temps du traitement extracorporel du sang au moins une fois, sur la base d'une baisse d'une concentration de substances urinaires dans le liquide de dialyse.

2. Dispositif selon la revendication 1, **caractérisé en ce que** l'unité de régulation/commande (100) est prévue et réalisée pour commander la source de rayonnement (2) de telle sorte que l'intensité du rayonnement (7) détectée par l'unité de détection (6, 10) reste constante pendant l'évolution dans le temps du traitement extracorporel du sang.

3. Dispositif selon la revendication 2, **caractérisé en ce que** l'ampérage avec lequel la source de rayonnement (2) est alimentée est évalué/utilisé pour la détermination de l'efficacité de dialyse/la dose de dialyse.

4. Dispositif selon la revendication 3, **caractérisé en ce que** l'efficacité de dialyse/la dose de dialyse est la valeur Kt/V, dans lequel K décrit la clairance, t la durée de thérapie et V le volume de répartition de substance urinaire dans le corps individualisé pour chaque patient, et l'unité de régulation/commande (100) est prévue et réalisée pour déterminer l'efficacité de dialyse/la dose de dialyse via la règle $Kt/V = \ln(I(t3)/I(t))$, dans lequel I décrit l'ampérage, t3 un moment donné et t la durée de thérapie.

5. Dispositif selon la revendication 3, **caractérisé en ce que** l'efficacité de dialyse/la dose de dialyse est la valeur Kt/V, dans lequel K décrit la clairance, t la durée de thérapie et V le volume de répartition de substance urinaire dans le corps individualisé pour chaque patient, et l'unité de régulation/commande (100) est prévue et réalisée pour déterminer l'efficacité de dialyse/la dose de dialyse via la règle $Kt/V = -\ln(-0{,}008*t+(I(t)/I(t3))+(4-3{,}5*(I(t)/I(t3))*UF/Wzu$, dans lequel I décrit l'ampérage, t3 un moment donné, t la durée de thérapie, UF le volume d'ultrafiltration et W le poids du patient (200).

6. Dispositif selon l'une quelconque des revendications 3 à 5, **caractérisé en ce que** l'ampérage déterminé est normalisé sur une valeur de température de référence.

7. Dispositif selon la revendication 1, dans lequel le dispositif présente une unité de détection (9) supplémentaire qui est prévue et réalisée pour détecter en tant qu'unité de détection de référence (9) l'intensité du rayonnement émis par la source de rayonnement, **caractérisé en ce que** le processus thérapeutique présente au moins une phase d'ajustement et au moins une phase de mesure, dans lequel l'unité de régulation/commande (100) réduit l'intensité du rayonnement émis (7) lors de l'évolution dans le temps du traitement extracorporel du sang au moins une fois lors de la phase d'ajustement et l'unité de régulation/commande (100) commande la source de rayonnement (2) par le biais de l'adaptation de l'ampérage de telle sorte que l'intensité du rayonnement (7) détectée par l'unité de détection de référence (9) reste constante pendant la phase de mesure et l'intensité du rayonnement (7) au niveau de l'unité de détection de mesure (10) destinée à la détermination de l'efficacité de dialyse/la dose de dialyse est évaluée pendant la phase de mesure.

**8.** Dispositif selon la revendication 7, **caractérisé en ce que** la phase d'ajustement et/ou la phase de mesure sont déclenchées par le biais de paramètres de thérapie.

**9.** Dispositif selon la revendication 7, **caractérisé en ce que** la phase d'ajustement et la phase de mesure sont effectuées à des moments définis du traitement extracorporel du sang.

**10.** Dispositif selon l'une quelconque des revendications 7 à 9, **caractérisé en ce qu'**une turbidité de la cuvette (3) est déterminée par le biais d'une modification de rapport entre l'intensité du rayonnement (7) détectée au niveau de l'unité de détection de mesure (10) et l'intensité du rayonnement (7) détectée au niveau de l'unité de détection de référence (9).

**11.** Dispositif selon l'une quelconque des revendications 1 à 10, **caractérisé en ce que** les ampérages et la durée avec lesquels la source de rayonnement (2) est alimentée avec les ampérages sont enregistrés et une prévision de la durée de vie (restante) de la source de rayonnement (2) est effectuée au moyen de ces valeurs enregistrées.

**12.** Dispositif selon l'une quelconque des revendications 1 à 11, **caractérisé en ce que** la cuvette (3) est disposée entre la source de rayonnement (2) et l'unité de détection de mesure (10).

Fig. 1

Fig. 2

Fig. 3

Fig. 4

Fig. 5

Fig. 6

Fig. 7

Fig. 8

Fig. 9

Fig. 10

Fig. 11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2397167 A1
- WO 2013186357 A1
- DE 102011008482 A1
- EP 2163271 A

**In der Beschreibung aufgeführte Nicht-Patentliteratur**

- **UHLIN,F ; LINDBERG, LG ; MAGNUSSON M.** *Am J Kidney Dis,* Mai 2003, vol. 41 (5), 1026-36